# FASCICULE DE BREVET EUROPEEN

(11) **EP 0 735 879 B1**
(45) Date de publication et mention de la délivrance du brevet: **12.03.2003**
(21) Numéro de dépôt: 95904579.0
(22) Date de dépôt: 21.12.1994
(51) Int. Cl.: A61K 31/55, A61K 47/48

(54) **UTILISATION DE CYCLODEXTRINES AMINEES POUR LA SOLUBILISATION AQUEUSE DES DIBENZAZEPINES UTILISABLES COMME AGENT ANTI-EPILEPTIQUE**
VERWENDUNG VON AMINIERTEN CYCLODEXTRINEN ZUM LÖSLICHMACHEN VON ALS ANTIEPILEPTIKA ANZUWENDENDEN DIBENZAZEPINDERIVATEN
USE OF AMINO CYCLODEXTRINS FOR THE AQUEOUS SOLUBILISATION OF DIBENZAZEPINES FOR USE AS AN ANTIEPILEPTIC AGENT.

(30) Priorité: 22.12.1993 FR 9315471
(43) Date de publication de la demande: 09.10.1996
(73) Titulaire: COMMISSARIAT A L'ENERGIE ATOMIQUE, 75015 Paris (FR)
(72) Inventeur: DJEDAINI-PILARD, Florence, F-91150 Etampes (FR); PERLY, Bruno, F-78320 Le-Mesnil-St.-Denis (FR)
(74) Mandataire: Audier, Philippe André
(86) Numéro de dépôt international: FR9401503
(87) Numéro de publication internationale: WO95017191

(56) Documents cités:
- EP-A- 0 435 826
- WO-A-90/02141
- WO-A-91/04026
- WO-A-91/13100
- WO-A-93/10794

## Description

La présente invention a pour objet un procédé de solubilisation dans un milieu aqueux de composés chimiques hydrophobes utilisables comme agent anti-épileptique.

De façon plus précise, elle concerne un procédé permettant de solubiliser dans des milieux aqueux des composés hydrophobes par inclusion de ces composés dans une cyclodextrine adaptée.

Les cyclodextrines ou cyclomaltooligosaccharides sont des composés d'origine naturelle formés par l'enchaînement de 6, 7 ou 8 unités glucose liées en α-1,4. De nombreux travaux ont montré que ces cyclodextrines pouvaient former des complexes d'inclusion avec des molécules hydrophobes et permettre ainsi la solubilisation de ces molécules dans des milieux aqueux. De nombreuses applications ont été proposées pour tirer profit de ce phénomène, en particulier dans le domaine pharmaceutique, comme il est décrit par D. Duchêne dans l'ouvrage intitulé "Cyclodextrins and their industrial uses", chapitre 6, pages 213 à 257, Editions de Santé, 1987). Des compositions pharmaceutiques utilisant ces cyclodextrines ont d'ailleurs été commercialisées au Japon, en Italie et plus récemment en France, par exemple par Pierre Fabre Médicament pour le Brexin® qui est un complexe d'inclusion du Piroxicam dans la β-cyclodextrine.

Parmi les cyclodextrines utilisables, la β-cyclodextrine qui comporte 7 unités glucose, est la plus adaptée au niveau de la taille de sa cavité et la moins chère des trois, mais son utilisation pose certains problèmes, car elle est moins soluble que les autres cyclodextrines et présente un caractère hémolytique.

Aussi, on a envisagé d'améliorer les propriétés de la β-cyclodextrine en la modifiant chimiquement pour la rendre plus adaptée. Plusieurs solutions ont été envisagées et ont conduit à l'utilisation de dérivés méthylés ou de dérivés hydroxyalkylés.

Les dérivés méthylés sont beaucoup plus solubles que la cyclodextrine d'origine et ils possèdent de bonnes propriétés de solubilisation de composés organiques hydrophobes, en particulier dans le cas de la 2,6-diméthyl-β-cyclodextrine. Néanmoins, ces dérivés méthylés, outre qu'ils sont difficiles à obtenir à l'état pur, sont inutilisables pour des applications pharmaceutiques, en particulier pour les formes injectables, en raison de leur très fort caractère hémolytique.

Les dérivés hydroxyalkylés, développés en particulier par Janssen, par exemple les hydroxypropyl-cyclodextrines présentent une très forte solubilité dans l'eau et sont peu hémolytiques. Toutefois, leur utilisation reste difficile en raison de leur extrême hétérogénéité chimique ; de plus, les substitutions peuvent limiter la formation de complexes d'inclusion par effet de gêne stérique, si bien qu'aucune application pharmaceutique n'a encore été mise au point avec ces dérivés.

La présente invention a précisément pour objet un procédé de solubilisation de composés chimiques hydrophobes utilisant des dérivés aminés de cyclodextrines, qui permettent de pallier ces inconvénients.

Selon l'invention, le procédé de solubilisation dans un milieu aqueux d'un composé chimique hydrophobe choisi dans le groupe des dibenzapépines consiste à combiner le composé chimique hydrophobe avec une cyclodextrine aminée de formule : 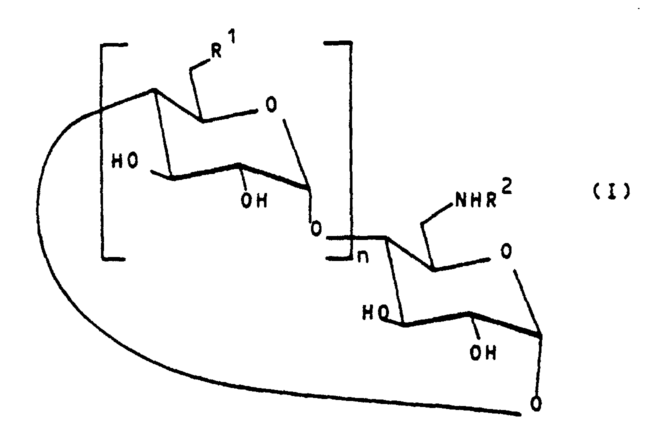 dans laquelle les R¹, qui peuvent être identiques ou différents, représentent OH ou NHR², R² représente un atome d'hydrogène ou un groupe alkyle et n est égal à 5, 6 ou 7,
pour former avec celle-ci un complexe d'inclusion soluble dans l'eau.

L'utilisation dans ce procédé de dérivés aminés répondant à la formule précitée présente l'avantage d'améliorer la solubilité, la stabilité et la biodisponibilité, sous diverses formes d'administration, du composé hydrophobe, en particulier pour des applications pharmaceutiques.

De préférence dans l'invention, on utilise un dérivé monoaminé de cyclodextrine, c'est-à-dire que dans la formule I précitée, tous les R¹ représentent OH. Le substituant aminé NHR² est de préférence NH₂; toutefois, on peut aussi utiliser comme groupe aminé des groupes monoalkylamino. Généralement, les groupes alkyle utilisés pour R² sont des groupes alkyle inférieurs ayant de 1 à 4 atomes de carbone.

Bien que dans l'invention, on préfère utiliser la β-cyclodextrine, c'est-à-dire le dérivé de formule (I) avec n=6, on peut aussi utiliser le dérivé aminé de l'α-cyclodextrine (n=5) ou le dérivé aminé de la γ-cyclodextrine (n=7).

Les composés chimiques hydrophobes susceptibles d'être solubilisés dans des milieux aqueux au moyen de ces cyclodextrines aminées sont des dibenzazépines qui sont des agents anti-épileptiques, utiles pour le traitement de l'épilepsie psychomotrice.

A titre d'exemple de tels composés, on peut citer la carbamazépine.

L'invention concerne également les complexes d'inclusion d'une cyclodextrine aminée répondant à la formule (I) précitée avec un composé chimique hydrophobe choisi dans le groupe des dibenzazépines, en particulier les agents anti-épileptiques.

Dans ces complexes d'inclusion, on préfère également que la cyclodextrine de formule (I) soit une cyclodextrine monoaminée, en particulier la β-cyclo-dextrine monoaminée.

Ces complexes d'inclusion peuvent être préparés par des procédés classiques, par exemple en ajoutant à une solution ou à une suspension de la cyclodextrine aminée de formule (I) utilisée, une solution du composé hydrophobe dans un solvant organique approprié, par exemple l'acétone. On peut ensuite isoler le complexe d'inclusion ainsi formé par lyophilisation.

L'invention a encore pour objet une composition pharmaceutique comprenant un complexe d'inclusion d'une cyclodextrine aminée de formule (I) et d'un composé chimique hydrophobe choisi dans le groupe des dibenzazépines, avec un véhicule pharmaceutiquement acceptable.

Ces compositions pharmaceutiques qui peuvent être administrées par voie orale ou parentérale, sont par exemple des solutions, des poudres, des suspensions, etc, en particulier des solutions injectables.

Les cyclodextrines aminées utilisées dans le procédé de l'invention peuvent être obtenues sous forme de chlorhydrate de très haute pureté, à partir des cyclodextrines naturelles par un procédé comprenant trois étapes.

Contrairement aux dérivés hydroxypropylés des cyclodextrines, ces dérivés aminés se présentent sous forme d'une espèce chimique unique qui est parfaitement caractérisée par des méthodes physico-chimiques classiques.

Par rapport aux dérivés méthylés qui présentent des propriétés de solubilisation comparables, elles ont l'avantage d'être considérablement moins hémolytiques.

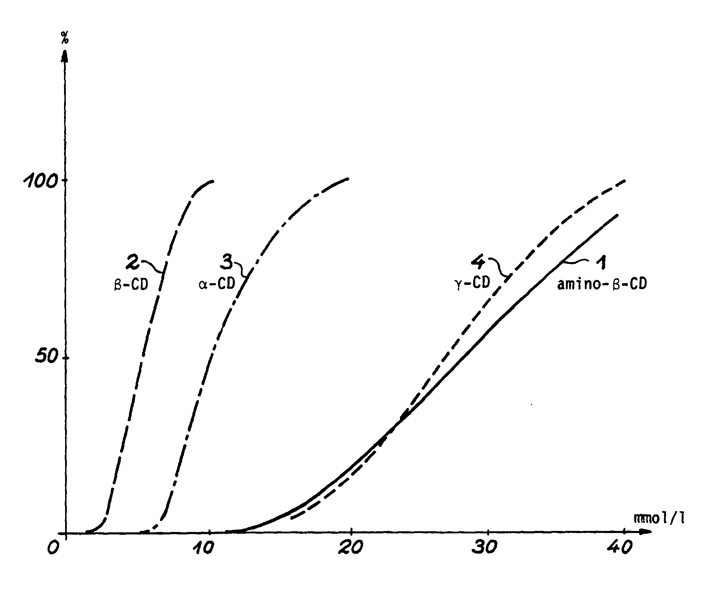

D'autres caractéristiques et avantages de l'invention apparaîtront mieux à la lecture des exemples suivants donnés bien entendu à titre illustratif et non limitatif, en référence au dessin annexé.

La figure annexée est un diagramme représentant les pourcentages d'hémolyse induite par différentes cyclodextrines en fonction de leur concentration (en mmol/l).

### Exemple 1 : Préparation de l'amino-β-cyclodextrine.

Ce dérivé de cyclodextrine répond à la formule (I) avec tous les R¹ représentant OH, R² représentant un atome d'hydrogène et n étant égal à 6.

On la prépare de la façon suivante.

### Synthèse de la mono-6-tosyl-β-cyclodextrine.

60 g de cyclomaltoheptaose (52,8 mmol) sont suspendus dans 500 ml d'eau distillée. On ajoute goutte à goutte 6,57 g (164 mmol) de soude caustique dissoute dans 20 ml d'eau sur 5 minutes avec forte agitation magnétique. A la solution limpide obtenue sont ajoutés 10,08 g (52,9 mmol) de chlorure de p-toluène sulfonyle (chlorure de tosyle) dans 30 ml d'acétonitrile goutte à goutte sur 10 minutes. Après 2 h d'agitation à température ambiante, le précipité formé est éliminé par filtration et le filtrat est conservé 48 h à 4°C. Le précipité est isolé par filtration sous vide, lavé par 50 ml d'eau glacée et recristallisé immédiatement dans l'eau bouillante. Après une nuit à 4°C, le précipité est filtré et séché sous vide à 30°C. On obtient ainsi 7,5 g (12 %) d'un composé pur conforme aux spécifications.

### Synthèse de la mono-6-azido-β-cyclodextrine.

Dans un ballon de 500 ml surmonté d'un réfrigérant, on introduit 15 g (11,6 mmol) de la tosyl cyclodextrine précédemment décrite en suspension dans 200 ml d'eau distillée. On ajoute à température ambiante et sous agitation 7,65 g d'azoture de lithium LiN₃ dans 40 ml d'eau. On porte le mélange à reflux (la solution devient limpide) pendant 4 heures et on laisse sous agitation à température ambiante pendant 18 heures. Le produit est précipité par addition de 150 ml d'acétone, maintenu dans la glace pendant 2 heures et isolé par filtration et lavage à l'acétone. Le composé blanc est lavé par 3 fois 20 ml d'éthanol bouillant pour éliminer les traces d'azoture de lithium et séché sous vide. On obtient 13,5 g (83 %) de monoazido-β-cyclodextrine pure.

### Synthèse de la mono-6-amino-β-cyclodextrine.

A 10 g (8,6 mmol) de mono-azido-β -cyclodextrine dans 120 ml de diméthylformamide (DMF), on ajoute goutte à goutte et sous agitation à température ambiante 9,06 g (4 équivalents) de triphénylphospine dissoute dans 30 ml de DMF. Le mélange réactionnel est maintenu une heure sous agitation, refroidi dans la glace et traité par 20 ml d'ammoniaque à 20 %. On laisse 18 heures sous agitation à température ambiante et on élimine le solvant sous pression réduite à 30°C. Le résidu est repris par 50 ml d'eau sous agitation. Le précipité de triphényl phosphine et de l'oxyde correspondant est éliminé par filtration et la solution est traitée par 150 ml d'acétone. Après une nuit à 4°C, le précipité est isolé, lavé à l'acétone et séché sous vide à 30°C. On obtient 9,6 g de mono-amino-β-cyclodextrine.

### Purification finale.

L'amino-cyclodextrine contient toujours une faible proportion de cyclodextrine non modifiée provenant de l'étape de tosylation. Cette dernière peut être éliminée facilement (cette purification est très importante) par une chromatographie sur résine échangeuse d'ions comme suit :

Une solution de mono-amino-β-cyclodextrine (1,5 g) dans 10 ml d'eau est appliquée à une colonne contenant 140 ml de résine Lewatit SP1080 forme H⁺ (Merck) en suspension dans l'eau. Après lavage par 400 ml d'eau (permettant d'éliminer la cyclodextrine non modifiée qui n'est pas retenue), la mono-amino-β -cyclodextrine est éluée par une solution aqueuse d'ammoniaque à 6 %. 100 ml de l'éluat basique sont collectés, concentrés sous vide à 30°C. Les dernières traces d'ammoniaque sont éliminées par évaporation sous vide en présence de 20 ml d'eau. Le résidu est repris dans le minimum d'eau et précipité par 150 ml d'acétone, maintenu à 4°C pendant une nuit, isolé par filtration, lavé à l'acétone et séché sous vide à 30°C. On obtient ainsi 1,15 g de composé final totalement débarrassé de la cyclodextrine d'origine.

Après dissolution dans l'eau et ajustement à pH ≅ 7 par HCl, la solution est lyophilisée.

On obtient donc cette amino-β-cyclodextrine sous la forme de son chlorhydrate.

La solubilité dans l'eau du composé obtenu est de 200 g/l à 20°C au lieu de 18 g/l pour la β-cyclo-dextrine naturelle, ce qui est comparable à la solubilité de la γ-cyclodextrine. En solution, de par son caractère de base faible, le chlorhydrate conduit à un pH proche des valeurs physiologiques (pH=6,8 dans le cas d'une solution à 1 % en poids du chlorhydrate dans de l'eau). Il agit ainsi comme un tampon.

Les propriétés hémolytiques du chlorhydrate ont été testées en mettant en contact 0,4 ml d'une suspension d'érythrocytes humains avec 4 ml de la solution du chlorhydrate dans 10 mmol d'un tampon phosphate isotonique à un pH de 7,4, pendant 30 minutes à 37°C.

Les résultats obtenus exprimés sous la forme du pourcentage d'hémolyse en fonction de la concentration en cyclodextrine de la solution sont donnés sur la figure annexée.

Sur cette figure, la courbe 1 se rapporte au chlorhydrate d'amino-β-cyclodextrine. Les courbes 2, 3 et 4 illustrent à titre comparatif les résultats obtenus dans les mêmes conditions avec une solution de β-cyclodextrine (β-CD, courbe 2), d'α-cyclodextrine (α-CD, courbe 3) et de γ-cyclodextrine (γ-CD, courbe 4). Au vu de cette figure, on remarque que le dérivé aminé de la β-cyclodextrine a un effet hémolytique bien inférieur à celui de la β-cyclo-dextrine et de l'α-cyclodextrine. En effet, la β -cyclodextrine induit 50 % d'hémolyse à une concentration de 5 mmol/l, c'est-à-dire de l'ordre de 6 g/l, alors que l'amino-β-cyclodextrine ne parvient à un tel effet qu'à des concentrations très supérieures, de l'ordre de 30 mmol/l, soit 35 g/l.

### Exemple 2 : Préparation d'un complexe d'inclusion de l'amino β-cyclodextrine et de carbamazépine

La carbamazépine est un composé répondant à la formule :

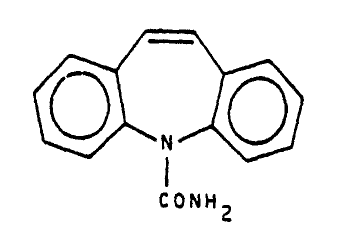

C'est un composé anti-épileptique (Merck Index 1783) de solubilité très limitée dans l'eau pure (12 µg/ml à 25°C). La disponibilité de formes injectables solubles pourrait permettre le traitement d'urgence de l'épilepsie avec ce composé.

On prépare un complexe d'inclusion de cette molécule avec la cyclodextrine monoaminée de l'exemple 1 en suivant le mode opératoire suivant :
On dissout 10 µmol de l'amino-β-cyclodextrine (chlorhydrate préparé dans l'exemple 1) dans 1 ml d'eau pure (eau apyrogène pour injection) et on ajoute 5 µmol de carbamazépine sous forme d'une solution concentrée à 50 mmol/1 dans l'acétone. On élimine l'acétone par passage d'azote pendant 10 min. et on lyophilise la solution.

On obtient ainsi un complexe d'inclusion.

### Exemple 3.

Dans cet exemple on évalue la solubilisation de la carbamazépine par différents dérivés de cyclodextrines dans des conditions standardisées. Les mesures de solubilité sont réalisées à 25°C en présence de la cyclodextrine considérée à la concentration de 25 mmol/l, le pH de la solution étant ajusté dans tous les cas à 7,4 pour simuler autant que posible les conditions physiologiques.

Les résultats obtenus et les cyclodextrines (CD) utilisées sont donnés dans le tableau 1 qui suit.

**Tableau 1**

| Cyclodextrine (25MM) | Carbamazépine |
|---|---|
| α-CD | < 8.10⁻⁵/M |
| β-CD | 3,6mM |
| γ-CD | 1,2mM |
| HPBCD ¹⁾ | 1,8mM |
| β-CD-NH₂ ²⁾ | 12 mM |
| Tampon Phosphate 7,4 | < 7.10⁻⁵ M |
| Eau pure | < 5.10⁻⁵ M |

| | |
|---|---|
| 1) Le composé HPBCD est l'hydroxypropyl-β-cyclodextrine. | |
| 2) β-CD-NH₂ = mono-6-amino-β-cyclodextrine de l'exemple 1. | |

Dans ce tableau, on a donné également à titre comparatif les solubilités de la carbamazépine dans l'eau pure et dans un tampon phosphate.

Au vu des résultats de ce tableau, on constate que l'amino-β-cyclodextrine de l'exemple 1 permet d'améliorer de façon importante la solubilité en milieu aqueux de la carbamazépine.

Des études de toxicité aigüe, de tolérance oculaire et de pouvoir mutagène de l'amino β-CD utilisée dans l'invention (ex 1) ont par ailleurs montré que :
1) - cette cyclodextrine n'entraîne aucune mortalité chez le rat Sprague-Dawley mâle et femelle lorsqu'elle est injectée par voie intraveineuse sous forme de chlorhydrate à la dose de 2g/kg de poids corporel;
2) - cette cyclodextrine n'induit aucune activité mutagène dans des souches d'Escherichia Coli et de Salmonella typhimurium selon le test de AMES ; et
3) - cette cyclodextrine se situe dans la classe 1 "produits faiblements irritants" pour la tolérance oculaire aigüe/évaluée par la méthode PREDISAFE.

De ce fait, l'utilisation selon l'invention de la mono-6-amino-β-cyclodextrine pour solubiliser la carbamazépine est très intéressante car il s'agit du premier moyen permettant de conditionner un anti-épileptique sous forme injectable, ce qui permet les soins en cas d'urgence.

## Revendications

1. Procédé de solubilisation dans un milieu aqueux d'un composé chimique hydrophobe choisi dans le groupe des dibenzazépines, **caractérisé en ce qu'**il consiste à combiner le composé chimique hydrophobe avec une cyclodextrine aminée de formule : 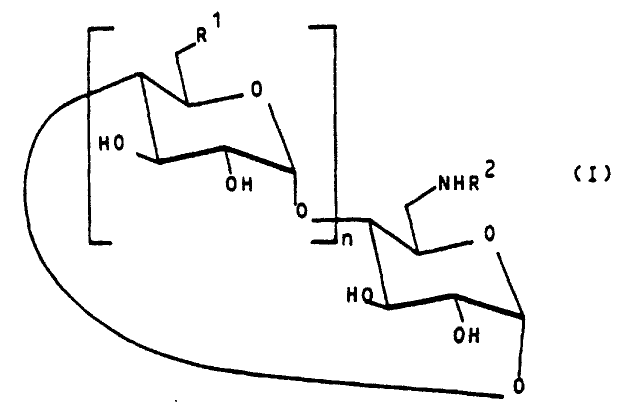 dans laquelle les R¹, qui peuvent être identiques ou différents, représentent OH ou NHR², R² représente un atome d'hydrogène ou un groupe alkyle et n est égal à 5, 6 ou 7,
pour former avec celle-ci un complexe d'inclusion soluble dans l'eau.

2. Procédé selon la revendication 1, **caractérisé en ce que** tous les R¹ représentent OH.

3. Procédé selon l'une quelconque des revendications 1 et 2, **caractérisé en ce que** R² est un atome d'hydrogène.

4. Procédé selon l'une quelconque des revendications 1 à 3, **caractérisé en ce que** n est égal à 6.

5. Procédé selon l'une quelconque des revendications 1 à 4, **caractérisé en ce que** le composé hydrophobe est la carbamazépine.

6. Complexe d'inclusion d'une cyclodextrine aminée de formule : 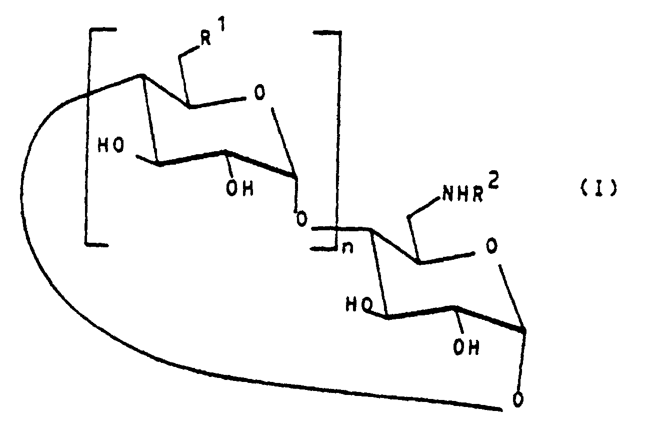 dans laquelle les R¹, qui peuvent être identiques ou différents, représentent OH ou NHR², R² représente un atome d'hydrogène ou un groupe alkyle et n est égal à 5, 6 ou 7,
avec un composé chimique hydrophobe choisi dans le groupe des dibenzazépines.

7. Complexe d'inclusion selon la revendication 6, **caractérisé en ce que** tous les R¹ représentent OH.

8. Complexe d'inclusion selon la revendication 7, **caractérisé en ce que** R² représente un atome d'hydrogène.

9. Complexe selon l'une quelconque des revendications 6 à 8, **caractérisé en ce que** n est égal à 6.

10. Complexe selon l'une quelconque des revendications 6 à 9, **caractérisé en ce que** le composé chimique hydrophobe est un agent anti-épileptique.

11. Complexe selon la revendication 10, **caractérisé en ce que** l'agent anti-épileptique est la carbamazépine.

12. Composition pharmaceutique, **caractérisée en ce qu'**elle comprend un complexe d'inclusion d'une cyclodextrine selon l'une quelconque des revendications 6 à 11 avec un véhicule pharmaceutiquement acceptable.

## Patentansprüche

1. Verfahren zur Solubilisierung einer hydrophoben chemischen Verbindung aus der Gruppe der Dibenzazepine in einem wässrigen Medium, **dadurch gekennzeichnet, dass** man die hydrophobe chemische Verbindung mit einem Aminocyclodextrin der Formel kombiniert: 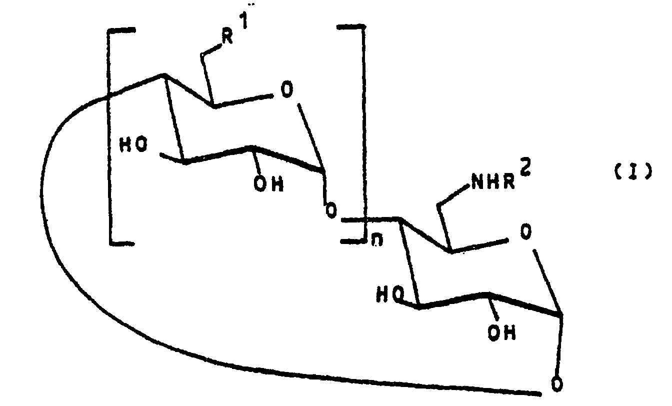 in der die Reste R¹, die gleich oder verschieden sein können, für OH oder NHR² stehen, worin R² ein Wasserstoffatom oder eine Alkylgruppe darstellt, und n für die Zahl 5, 6 oder 7 steht,
um mit dieser einen in Wasser löslichen Einschlusskomplex zu bilden.

2. Verfahren nach Anspruch 1, **dadurch gekennzeichnet, dass** alle Reste R¹ für OH stehen.

3. Verfahren nach einem der Ansprüche 1 und 2, **dadurch gekennzeichnet, dass** R² ein Wasserstoffatom darstellt.

4. Verfahren nach einem der Ansprüche 1 bis 3, **dadurch gekennzeichnet, dass** n für die Zahl 6 steht.

5. Verfahren nach einem der Ansprüche 1 bis 4, **dadurch gekennzeichnet, dass** es sich bei der hydrophoben Verbindung um Carbamazepin handelt.

6. Einschlusskomplex eines Aminocyclodextrins der Formel 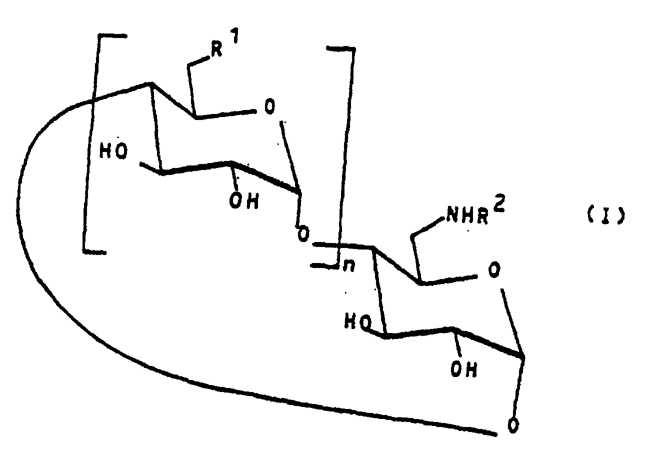 in der die Reste R¹, die gleich oder verschieden sein können, für OH oder NHR² stehen, wobei R² ein Wasserstoffatom oder eine Alkylgruppe darstellt, und n für die Zahl 5, 6 oder 7 steht,
mit einer hydrophoben chemischen Verbindung aus der Gruppe der Dibenzazepine.

7. Einschlusskomplex nach Anspruch 6, **dadurch gekennzeichnet, dass** alle Reste R¹ für OH stehen.

8. Einschlusskomplex nach Anspruch 7, **dadurch gekennzeichnet, dass** R² ein Wasserstoffatom darstellt.

9. Komplex nach einem der Ansprüche 6 bis 8, **dadurch gekennzeichnet, dass** n für die Zahl 6 steht.

10. Komplex nach einem der Ansprüche 6 bis 9, **dadurch gekennzeichnet, dass** die hydrophobe chemische Verbindung ein antiepileptisches Agens darstellt.

11. Komplex nach Anspruch 10, **dadurch gekennzeichnet, dass** das antiepileptische Agens das Carbamazepin ist.

12. Pharmazeutische Zusammensetzung, **dadurch gekennzeichnet, dass** sie einen Einschlusskomplex eines Cyclodextrins nach einem der Ansprüche 6 bis 11 zusammen mit einem pharmazeutisch akzeptablen Vehiculum enthält.

## Claims

1. Process for the solubilization in an aqueous medium of a hydrophobic chemical compound chosen from within the group of dibenzazepines, **characterized in that** it consists of combining the hydrophobic chemical compound with an aminocyclodextrin of formula: 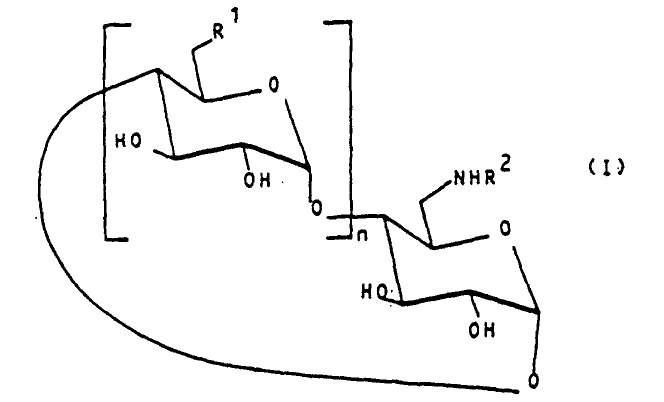 in which the R¹, which can be the same or different, represent OH or NHR², R² represents a hydrogen atom or an alkyl group and n is equal to 5, 6 or 7, in order to form therewith a water-soluble inclusion complex.

2. Process according to claim 1, **characterized in that** all the R¹ represent OH.

3. Process according to either of the claims 1 and 2, **characterized in that** R² is a hydrogen atom.

4. Process according to any one of the claims 1 to 3, **characterized in that** n is equal to 6.

5. Process according to any one of the claims 1 to 4, **characterized in that** the hydrophobic compound is carbamazepine.

6. Inclusion complex of an aminocyclodextrin of formula: 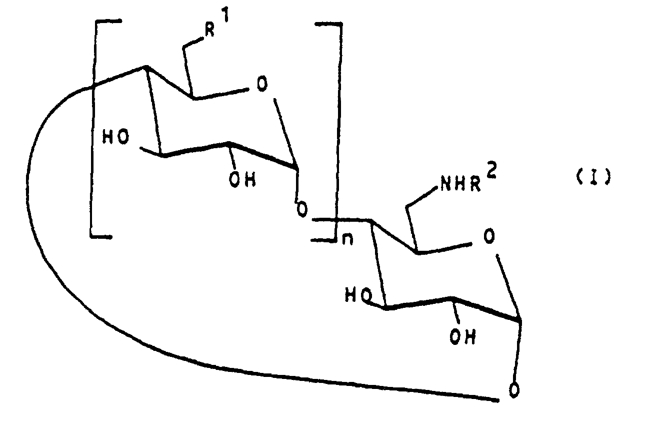 in which the R¹, which can be the same or different, represent OH or NHR², R² represents a hydrogen atom or an alkyl group and n is equal to 5, 6 or 7, with a hydrophobic chemical compound chosen from within the group of dibenzazepines.

7. Inclusion complex according to claim 6, **characterized in that** all the R¹ represent OH.

8. Inclusion complex according to claim 7, **characterized in that** R² represents a hydrogen atom.

9. Complex according to any one of the claims 6 to 8, **characterized in that** n is equal to 6.

10. Complex according to any one of the claims 6 to 9, **characterized in that** the hydrophobic chemical compound is an anti-epileptic agent.

11. Complex according to claim 10, **characterized in that** the anti-epileptic agent is carbamazepine.

12. Pharmaceutical composition, **characterized in that** it comprises an inclusion complex of a cyclodextrin according to any one of the claims 6 to 11 with a pharmaceutically acceptable vehicle.
